(19) **Europäisches Patentamt European Patent Office Office européen des brevets**

(11) **EP 1 250 084 B1**

(12) **FASCICULE DE BREVET EUROPEEN**

(45) Date de publication et mention de la délivrance du brevet:
**09.11.2016 Bulletin 2016/45**

(21) Numéro de dépôt: **01907693.4**

(22) Date de dépôt: **25.01.2001**

(51) Int Cl.:
***A61B 5/00*** (2006.01)

(86) Numéro de dépôt international:
**PCT/FR2001/000234**

(87) Numéro de publication internationale:
**WO 2001/054571 (02.08.2001 Gazette 2001/31)**

(54) **SYSTEME POUR LE SUIVI A DISTANCE DE PATIENTS**

SYSTEM ZUR FERNÜBERWACHUNG VON PATIENTEN

REMOTE PATIENT MONITORING SYSTEM

(84) Etats contractants désignés:
**AT BE CH CY DE DK ES FI FR GB GR IE IT LI LU MC NL PT SE TR**

(30) Priorité: **25.01.2000 FR 0000903**

(43) Date de publication de la demande:
**23.10.2002 Bulletin 2002/43**

(73) Titulaire: **Diatelic**
**54600 Villers-Lès-Nancy (FR)**

(72) Inventeurs:
• **HERVY, Robert**
  **F-54140 Jarville (FR)**
• **ROMARY, Laurent**
  **F-54270 Essey-les-Nancy (FR)**
• **CHARPILLET, François**
  **F-54506 Vandoeuvre-les-Nancy (FR)**
• **PIERREL, Jean-Marie**
  **F-54600 Villers-les-Nancy (FR)**
• **THOMESSE, Jean-Pierre**
  **F-54140 Jarville (FR)**
• **PETIT JEAN, Etienne**
  **54500 Vandoeuvre (FR)**
• **JEANPIERRE, Laurent**
  **F-54180 Houdemont (FR)**
• **DURAND, Pierre-Yves**
  **54000 Nancy (FR)**
• **CHANLIAU, Jacques**
  **F-54230 Neuves-Maisons (FR)**

(74) Mandataire: **Regimbeau**
  **20, rue de Chazelles**
  **75847 Paris Cedex 17 (FR)**

(56) Documents cités:
EP-A- 0 761 160     WO-A-96/28086
FR-A- 2 717 332     US-A- 5 438 983

• **KOENIG S ET AL: "UNSUPERVISED LEARNING OF PROBABILISTIC MODELS FOR ROBOT NAVIGATION" PROC. OF THE INTERNAT. CONF. ON ROBOTICS AND AUTOMATION,US,NEW YORK, IEEE, 22 avril 1996 (1996-04-22), pages 2301-2308, XP000772551 ISBN: 0-7803-2989-9 cité dans la demande**
• **PATENT ABSTRACTS OF JAPAN vol. 1995, no. 07, 31 août 1995 (1995-08-31) & JP 07 095963 A (TEIJIN LTD), 11 avril 1995 (1995-04-11) & JP 07 095963 A 11 avril 1995 (1995-04-11)**

**Description**

PRESENTATION GENERALE DE L'INVENTION

La présente invention est relative à un système pour le suivi à distance de patients.

On connaît déjà des systèmes de surveillance médicale à distance dans lesquels le patient est équipé de moyens par lesquels il transmet au médecin qui le suit des valeurs de paramètres physiologiques permettant un suivi quotidien du patient.

On pourra par exemple à cet égard se référer à la demande de brevet FR 2 717 332 et à la demande internationale WO 96/28086.

Dans le système décrit dans ces demandes, il est mis en oeuvre sur les données reçues du patient un traitement permettant au médecin de prendre facilement connaissance des données qui lui sont transmises (présentations sous forme de tableaux, de graphiques, de statistiques, etc.) et de mettre en évidence pour celui-ci l'évolution des paramètres physiologiques mesurés sur une certaine période de temps.

La demande internationale WO 96/28086 décrit un système de suivi de patient conforme au préambule de la revendication 1.

Un but de l'invention est de proposer un système de suivi à distance de patients qui est encore amélioré et constitue pour le médecin un véritable outil d'assistance apte à mettre en évidence les évolutions anormales de l'état d'un patient et ce avec une grande fiabilité.

Un autre but de l'invention, plus général, est de proposer un système permettant d'améliorer la qualité du suivi des patients, en même temps que leur confort, leur sécurité, leurs relations avec le monde médical et qui puisse également être utilisé pour une assistance à la recherche médicale.

Ainsi, l'invention propose un système de suivi de patients à domicile comportant des moyens d'interface chez le patient, des moyens d'interface chez un médecin, un serveur relié par un réseau aux moyens d'interface chez le patient et aux moyens d'interface chez le médecin, ledit serveur recevant des moyens d'interface chez le patient des valeurs de paramètres physiologiques mesurées par le patient ou par des capteurs et comportant des moyens pour mémoriser un historique, correspondant à une certaine période de temps, sur les valeurs de paramètres physiologiques qui lui sont transmises, ledit serveur comportant en outre des moyens pour mettre en oeuvre sur les valeurs de paramètres physiologiques transmises par le patient un traitement en vue de leur présentation et de la présentation de leur évolution sur les moyens d'interface chez le médecin, lesdits moyens mettant en oeuvre sur les dernières valeurs de paramètres physiologiques qui sont transmises au serveur un traitement comparant l'état défini par ces différentes valeurs de paramètres physiologiques à des états d'alerte définis en fonction de l'historique mémorisé pour le patient et comportant des moyens pour transmettre les résultats de ce traitement aux moyens d'interface chez le médecin, caractérisé en ce que les moyens de traitement comprennent en outre des moyens pour calculer une probabilité d'observer une valeur donnée pour un paramètre physiologique donné et un temps donné, en supposant connu un état dans lequel se trouve le patient, de la manière suivante :

$$P(v_{i,t}|s) = \sum_{o \in O(i)} f_{s,o}(v_{i,t}) p_i(o|s)$$

où

$v_{i,t}$ est la valeur observée pour le paramètre physiologique $i$ à un instant $t$,

$O_{(i)}$ est l'ensemble des observations possibles pour le paramètre $i$,

$f$ est une fonction qui donne une pondération comprise entre 0 et 1, qui dépend de la valeur du paramètre physiologique $i$ pour un état donné $s$ et dont les paramètres sont définis par le médecin ou dépendent de l'historique mémorisé pour le patient,

$p_i(o|s)$ est la probabilité d'observer o pour le paramètre $i$ en supposant que l'état du patient est $s$ "

en ce que le calcul de probabilité est effectué pour différents états possibles pour le patient, et en ce que les probabilités ainsi calculées pour les différents états possibles pour le patient sont transmises aux moyens d'interface chez le médecin.

Un tel système est avantageusement complété par les différentes caractéristiques suivantes prises seules ou selon toutes leurs combinaisons possibles :

- les moyens de traitement sont aptes à permettre au médecin de modifier les modélisations de probabilités déterminées pour les différents états physiologiques ;

- les moyens d'interface chez le médecin sont aptes à permettre à celui-ci de proposer des modifications sur les valeurs de probabilités calculées par les moyens de traitement, lesdits moyens de traitement comportant des moyens pour modifier les modélisations de probabilités déterminées pour les différents états physiologiques en fonction des valeurs modifiées proposées par le médecin ;
- les moyens de traitement déterminent la probabilité de se trouver dans un état donné pour une valeur de paramètre physiologique donnée en fonction du rapport entre d'une part la différence entre cette valeur de paramètre physiologique et une valeur de référence normale pour ce paramètre et d'autre part une tolérance ;
- pour au moins un paramètre physiologique, la valeur de référence est fonction de l'historique du patient ;
- lesdits moyens de traitement comparent les valeurs de paramètres physiologiques ou des valeurs d'autres paramètres déterminées en fonction de celles-ci à des valeurs seuils qui sont déterminées en fonction de l'historique mémorisé et, lorsqu'une telle valeur seuil est dépassée, transmettent un message d'alerte aux moyens d'interface chez le médecin et/ou chez le patient ;
- les moyens de traitement déterminent des valeurs de paramètres appelés paramètres de tendances qui sont fonction des dernières valeurs de paramètres physiologiques transmises par l'interface chez le patient et d'une partie de l'historique mémorisé qui précède juste dans le temps la transmission de ces dernières valeurs de paramètres physiologiques, comparent ces valeurs de paramètres de tendances à des valeurs seuils et, lorsqu'une telle valeur seuil est dépassée, transmettent un message d'alerte aux moyens d'interface chez le médecin et/ou chez le patient ;
- au moins une partie des dernières valeurs de paramètres physiologiques transmises par l'interface chez le patient sont en outre comparées à d'autres valeurs seuils indépendante de l'historique et en ce que la valeur d'au moins un paramètre de tendance est modifiée lorsque l'une de ces valeurs seuils est dépassée ;
- le serveur comporte des moyens pour, lorsqu'un patient cherche à se connecter, identifier ledit patient, interrompre la communication et se connecter aux moyens d'interface dudit patient ;
- les moyens d'interface chez le patient ne permettent à celui-ci de transmettre des informations à destination du médecin que lorsque le patient a rempli une fiche de paramètres quotidiens ;
- les moyens d'interface chez le patient comportent des moyens pour contrôler la cohérence des valeurs de paramètres physiologiques mesurées et transmises par le patient.

## PRESENTATION DES FIGURES

D'autres caractéristiques et avantages de l'invention ressortiront encore de la description qui suit. Cette description est purement illustrative et non limitative. Elle doit être lue en regard des dessins annexés sur lesquels :

- la figure 1 est une représentation schématique illustrant une architecture opérationnelle d'un système conforme à un mode de réalisation possible pour l'invention ;
- la figure 2 est un graphe sur lequel on a porté des courbes qui illustrent des fonctions utilisées par le serveur du système de la figure 1 ;
- la figure 3 et la figure 4 sont deux graphes illustrant deux modes de présentation possibles des résultats du traitement du serveur au niveau des moyens d'interface chez le médecin ;
- la figure 5 est un graphe sur lequel on a porté différentes courbes utilisées pour le calcul des probabilités.

## DESCRIPTION D'UN OU PLUSIEURS MODES DE REALISATION PARTICULIERS

### *Architecture générale*

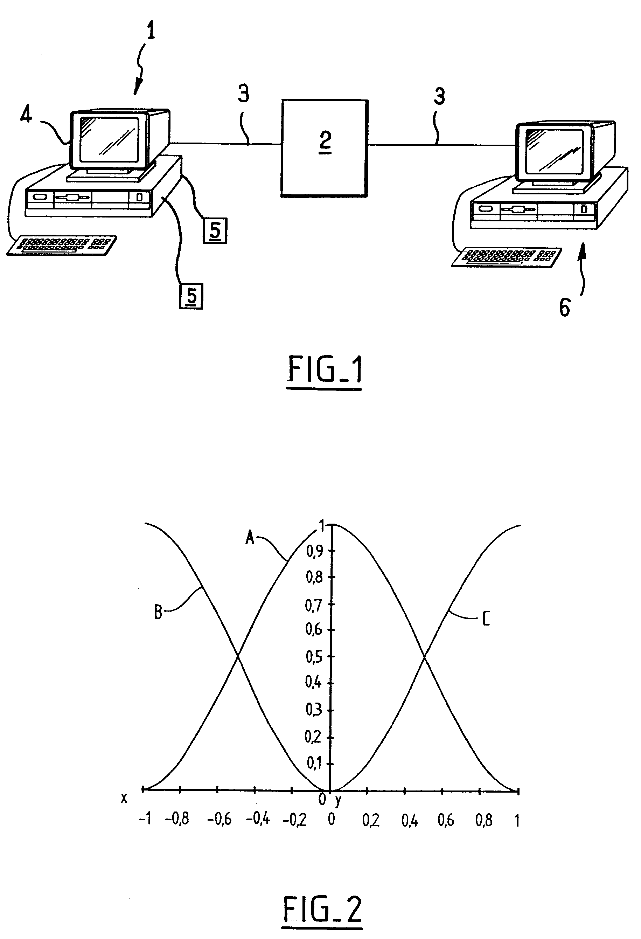

Le système qui est illustré sur la figure 1 comporte des moyens d'interface 1 disposés au domicile du patient, un serveur informatique 2 avec lequel les moyens 1 dialoguent par l'intermédiaire du réseau téléphonique, référencé par 3, et des moyens d'interface 6 disposés chez le médecin.

Les moyens 1 au domicile du patient sont constitués par un terminal informatique 4 qui est éventuellement relié à des capteurs 5 qui permettent d'enregistrer automatiquement des valeurs de paramètres physiologiques du patient.

Le patient échange par exemple quotidiennement avec le serveur 2 à partir des moyens 1.

A cet effet, lorsque le patient s'est connecté au serveur 2 par sa ligne téléphonique, il s'identifie en fournissant au serveur 2 un code d'identification, ainsi qu'un mot de passe.

La communication est alors interrompue et le patient est ré-appelé par le serveur 2.

Comme on l'aura compris, cette décomposition de la connexion en deux étapes, et notamment le fait que le serveur ré- appelle le patient après que celui-ci se soit identifié, permet de sécuriser l'utilisation du système.

Une fois la connexion ré-établie, un premier écran présente au patient les messages que son médecin a pu lui laisser.

Une fois que le patient a pris connaissance de ces messages, il lui est demandé de remplir une fiche de paramètres quotidiens dans laquelle il doit indiquer, pour les paramètres physiologiques dont les valeurs ne sont pas transmises automatiquement par les capteurs, les valeurs des paramètres physiologiques qu'il a pu lui-même mesurer.

Le terminal informatique 4 met alors en oeuvre un traitement permettant de contrôler la cohérence de la valeur introduite pour chaque paramètre.

Par exemple, il compare la valeur des paramètres à des valeurs seuils.

Lorsque l'ensemble des rubriques de la fiche sont remplies - et uniquement dans ce cas -, l'ensemble des valeurs de paramètres physiologiques mesurées est transmis au serveur 2.

Les moyens 6 sont quant à eux constitués par un simple terminal informatique, qui reçoit les résultats d'un traitement mis en oeuvre par le serveur 2.

Ce traitement est destiné à présenter au médecin les dernières valeurs de paramètres physiologiques mesurées et le cas échéant à détecter et mettre en évidence pour le médecin une éventuelle évolution anormale de ces valeurs de paramètres.

On notera que lorsqu'une telle évolution anormale est détectée, celle-ci est immédiatement signalée au patient pour qu'il puisse, de son propre fait, contacter les médecins qui le suivent.

On va maintenant décrire des exemples de traitement possibles dans le cas où le système est utilisé pour la surveillance de dialysés à domicile et notamment pour la surveillance de patients soumis à la dialyse péritonéale continue ambulatoire (DPCA).

Bien entendu, d'autres applications du système proposé par l'invention sont envisageables, l'application au suivi de patients soumis à la dialyse péritonéale continue ambulatoire ne se voulant qu'un exemple parmi d'autres possibles.

Le traitement mis en oeuvre par le serveur 2 est un traitement à système expert qui est personnalisé en fonction de l'historique du patient.

Notamment, le serveur 2 garde en mémoire les valeurs des paramètres physiologiques du patient et des données calculées à partir de celles-ci sur une durée qui peut être de seize jours.

### *Premier exemple de traitement*

Un traitement conforme à un premier exemple possible est un traitement qui d'une part met en oeuvre une détermination d'alertes sur des paramètres appelés tendances qui sont fonction de l'historique du patient et d'autre part met en oeuvre une détection d'alertes à partir de différentes règles de traitement sur les valeurs de paramètres physiologiques juste transmises par le patient.

Les paramètres de tendances sont par exemple l'hydratation, la température, la tension couchée et la tension debout, les valeurs de volume de drainage des poches.

Les valeurs de ces paramètres de tendances sont déterminées en fonction de l'historique récent, par exemple moyennées sur une durée de quelques jours, et comparées à un seuil de déclenchement d'alerte, qui est fonction des valeurs normales du patient.

On comprend que du fait que les paramètres de tendances sont calculés en prenant en compte non seulement les dernières valeurs reçues du patient, mais également les valeurs sur la durée correspondant à celle choisie pour l'historique récent, les paramètres de tendances ne vont générer des alertes que dans les cas où les paramètres physiologiques se seront écartés de leur valeur normale non pas uniquement ponctuellement à un instant donné, mais sur une certaine durée (celle de l'historique récent).

Ainsi, par exemple, si un patient a une température de 38°, ceci n'a pas de conséquence ponctuellement, mais devient inquiétant si cette température se maintient pendant un certain temps.

Le traitement proposé permet de déclencher un signal d'alerte permettant de mettre en évidence cette dérive "à long terme"

Par exemple, une tendance utilisée est une tendance hydratation.

La tendance hydratation est un nombre compris entre -99 et +99 qui reflète, lorsqu'il est positif le degré d'hyperhydratation et lorsqu'il est négatif le degré de déshydratation.

Lorsque l'hydratation est normale, la tendance est proche de zéro.

La tendance hydratation est initialisée à 0. Lorsque sa valeur absolue dépasse 100 une alerte « Majeure » est générée. Celle-ci sera indiquée au patient, et apparaîtra en priorité sur l'interface médecin. Ceci ne se produit que lorsque plusieurs symptômes corroborent un même diagnostic, ou qu'un même facteur se répète plusieurs jours de suite.

La tendance hydratation est incrémentée de +30 lorsque la règle relation-poids-hydratation est déclenchée, de -30 lorsque la règle relation-poids-déshydratation est déclenchée, -25 lorsque la règle différence-couché-debout est déclenchée.

Chaque jour la tendance hydratation est atténuée d'un facteur 1,5 et augmentée d'une valeur égale à 25 fois le rapport entre l'écart au poids sec divisé par 1,5. Cette valeur est significative lorsque l'écart au poids sec est supérieur à 1,5 KG (1,5 étant la tolérance sur le poids).

On notera que les valeurs normales qui sont utilisées sont elles-mêmes fonction de l'historique du patient sur une durée plus importante. Elles sont par exemple déterminées en calculant la valeur moyenne du paramètre considéré sur une durée de seize jours.

Par ailleurs, les valeurs de paramètres de tendances sont en outre modifiées, comme cela sera décrit plus loin, en fonction de l'état du patient tel qu'il résulte des valeurs de paramètres physiologiques transmises en dernier lieu par le patient, et notamment lors de détection d'états d'alerte sur ces paramètres.

Le traitement de détection des états d'alerte sur les valeurs de paramètres physiologiques transmises en dernier lieu met quant à lui en oeuvre les règles suivantes :

- *RELATION-POIDS-HYPERHYDRATATION :* cette première règle vérifie si le poids du patient dépasse son poids sec. La limite fixée par les médecins est de 1,5 Kg. Si cette limite est franchie, une alerte *"Hyperhydratation"* est déclenchée, et un malus de 30% est ajouté à la tendance *HYDRATATION*

- *RELATION-POIDS-DESHYDRATATION :* cette règle est la symétrique de la précédente. Elle vérifie quant à elle si le poids passe au dessous du poids sec. (une tolérance également de 1,5 Kg est acceptée.) Dans ce cas, une alerte *"Déshydratation"* est déclenchée, et un malus de 30% est retiré à la tendance *HYDRATATION.*

- *DIFFE-RENCE-CoucHE-DE-BOUT* : cette règle surveille la différence entre la tension du patient debout, et la tension du patient couché. En temps normal, cette différence est relativement réduite. Lorsque le patient commence à se déshydrater, cette valeur s'affole. Une limite arbitraire de 1,5 a été fixée. Le test est effectué sur la différence entre les tensions moyennes des deux positions (debout et couché). Si la limite est dépassée, une alerte *"Déshydratation"* est déclenchée, et un malus de 25% est amputé à la tendance *HYDRATATION.*

- *VARIATION-TENSION-COUCHE :* cette règle surveille les variations de la tension du patient couché. Ce test est effectué sur la moyenne des tensions systoliques et diastoliques, à laquelle on soustrait la valeur moyennée dans le temps pour cette valeur pour le patient. Lorsque l'on dépasse une limite arbitraire de 1,5, une alerte *"Modification rapide la tension couché"* est générée. Cependant, et contrairement aux autres règles de ce groupe, aucun malus n'est infligé aux tendances. En effet, il ne semble pas y avoir de lien direct avec l'état du patient, sauf peut-être que ce genre d'alerte indique qu'il ne va pas bien!

- *VARIATION-TENSION-DEBOUT* : cette règle est symétrique de la précédente. Elle vérifie quant à elle les variations de la tension du patient debout. Pour le reste, son comportement est strictement identique.

- *POCHE_1, POCHE_2, POCHE_3,* **et** *POCHE_4* : ces quatre règles vérifient le volume drainé par chacune des quatre poches du patient. Selon le type de la poche, le volume drainé doit rester à peu près constant, même si des variations importantes sont possibles. C'est pourquoi aucune alerte n'est attachée à ces règles. Cet dernières se chargent donc simplement de mettre à jour la tendance *POCHES* en fonction des volumes observés. Cette mise en forme se fait à partir de la formule suivante qui a été déterminée empiriquement :

$$(((\text{sortie de la poche} - \text{entrée de la poche}) - \text{valeur moyenne de ce type de poche})/200)* 15 ) = \text{Nouvelle valeur de paramètre de poche}$$

Après détermination de ces différents états d'alerte, le système met à jour les valeurs normales utilisées pour la gestion des paramètres de tendances, c'est à dire détermine les nouvelles valeurs normales à prendre en compte lors du prochain traitement, et ce en calculant de nouvelles valeurs moyennes sur l'ensemble de l'historique, ces nouvelles valeurs moyennes intégrant les dernières valeurs de paramètres physiologiques transmises par le patient.

*Deuxième exemple de traitement*

Un deuxième traitement par système expert possible va maintenant être décrit.

Ce traitement a été développé à partir d'un modèle de décision markovien partiellement observable (POMDP).

*Rappels théoriques.*

Classiquement, un modèle s'exprime sous forme d'un n-uplet < S, A, O, B, T, R, $\Pi$ > dans lequel :

- S représente l'ensemble fini des états de l'environnement que l'on cherche à modéliser. Lorsque ces états ne sont pas directement observables, un modèle des observations doit être défini. Ce modèle comprend un ensemble fini O des observations possibles et une fonction d'observation B qui a un état de S ou plus généralement à un couple (état, action) associe une distribution de probabilité sur les éléments de O. B(o|s, a) représente la probabilité d'observer $o \in O$ depuis l'état s (en ayant pris l'action a).
- A est l'ensemble fini des actions qui permettent d'influer sur le processus. Ces actions sont destinées à faire évoluer le système d'un état à un autre. Les actions ont un effet incertain qui est modélisé par la fonction de transition définie ci-dessous.
- O est l'ensemble fini des observations qui permet de caractériser le modèle.
- B est la fonction d'observation
- T est la fonction de transition qui définit la probabilité de passer de l'état s à l'état s' en effectuant l'action de A. : p(s' s,a)
- R est la fonction de récompense qui associe à chaque état, ou à chaque couple (état, action) un nombre qui exprime le degré de satisfaction de mettre le système dans l'état s, ou de choisir l'action a lorsqu'on est dans l'état s.
- $\Pi$ donne la distribution de probabilité initiale sur l'ensemble des états.

Un modèle exprimé sous cette forme peut être exploité par un système automatique pour répondre aux questions suivantes :

- étant donnée une séquence de vecteurs d'observation $(o_1, ...o_T)$ quelle est la probabilité que le système se trouve dans l'état s à l'instant T (diagnostic).
- étant donnés une séquence de vecteurs d'observation $(o_1, ...o_T)$ et un modèle $\lambda$, comment ajuste-t-on les paramètres $< B, T, \Pi >$ pour maximiser $p(o_1, ...o_T|\lambda)$ (apprentissage).
- étant donné une séquence de vecteurs d'observation qu'elle est l'action optimale à entreprendre pour que le système atteigne un état donné (recommandation d'actions).

Un tel modèle est par exemple du type de ceux décrits dans la publication :

- Koenig, R. G. Simmons dans « Unsupervised Learning of Probabilistic Models for Robot Navigation » publiés dans les actes de la conférence IEEE ICRA'96.

*Application d'un tel modèle au traitement proposé pour le suivi de l'hydratation.*

**Définition des éléments du modèle**

Les états de l'ensemble S utilisés sont au nombre de cinq : hydratation normale, déshydratation, hyper-hydratation, poids sec sous-estimé et poids sec surestimé.

Les observations de l'ensemble O sont quant à elles constituées, pour chaque paramètre physiologique des symboles qualificatifs O(i) suivants : conforme$_i$, inférieur-a-la-norme$_i$, supérieur-a-la-norme$_i$, où i est un indice qui selon sa valeur désigne l'un ou l'autre des paramètres physiologiques.

Les paramètres physiologiques considérés sont le poids, la tension, la tension orthostatique et le bilan des poches. lesquels paramètres sont calculables aisément à partir de la fiche qui est télétransmise quotidiennement.

Plus précisément, pour déterminer si le poids est conforme, inférieur à la norme, supérieur à la norme, on surveille la variation de la différence entre le poids du patient et le poids sec fixé par le médecin.

Notamment, on compare cette différence à des valeurs seuils inférieures ou supérieures qui sont par exemple de plus ou moins 1,5 kg.

Pour déterminer si la tension est conforme, inférieure à la norme ou supérieure à la norme, on surveille les variations de la différence entre la dernière tension transmise et une tension moyenne calculée sur l'historique mémorisé pour le patient.

Notamment, cette différence est comparée à des valeurs seuils qui sont par exemple de plus ou moins 1,2.

Egalement, pour déterminer si la tension orthostatique est conforme, inférieure à la norme, supérieure à la norme, on détermine la différence entre les tensions du patient lorsqu'il est couché et lorsqu'il est debout. La normale a été fixée à 0,5, la tolérance à 1,5.

Enfin, pour déterminer si le bilan des poches est conforme, inférieur à la norme ou supérieur à la norme, on détermine la différence entre le volume drainé par les poches du patient et un volume drainé moyen déterminé sur l'historique du patient.

La valeur normale est nulle, c'est à dire qu'en moyenne, un type de poches donne à peu près toujours les mêmes flux. La tolérance a été fixée à 60%. Cette tolérance est relativement forte, car trop de paramètres échappent à notre contrôle pour que cela soit vraiment fiable.

On notera que dans le cas des surveillances dans lesquelles on fait intervenir une moyenne sur l'historique du patient, cet historique est par exemple de quinze jours.

La fonction d'observation B représente quant à elle la probabilité d'observer, pour un paramètre physiologique i donné, l'un des symboles qualitatifs de l'ensemble O, connaissant l'état s du système.

On comprend, si l'on se réfère à la définition des symboles qualitatifs d'observation, que ces symboles ne sont pas directement accessibles à partir des données transmises au serveur.

Le système met donc en oeuvre un traitement permettant d'estimer la probabilité d'observer une valeur $V_{i,t}$ pour un paramètre physiologique i donné et un temps t donné, en supposant connu l'état dans lequel se trouve le système.

Cette probabilité s'exprime de la façon suivante :

$$v_{i,t.} : p(v_{i,t}|s) = \sum_{o \in o(i)} f_{s,o}(v_{i,t}) p_i(o|s)$$

où f est une fonction du type de celle illustrée sur la figure 2 qui donne une pondération comprise entre 0 et 1 et qui est fonction de la valeur du paramètre physiologique i pour un état s donné.

Par exemple, pour l'état conforme à la norme, cette fonction aura une évolution en fonction de la valeur du paramètre physiologique du type de celle illustrée sur la courbe A sur la figure 2.

Pour l'observation qualificative inférieure à la norme, la fonction aura la forme représentée par la courbe B sur la figure 2.

Pour l'observation qualificative supérieure à la norme, la fonction aura la forme de la courbe C sur la figure 2.

Ces différentes courbes A, B, C sont en l'occurrence des sigmoïdes.

Les courbes A, B et C sont des courbes définies en fonction du rapport (valeur mesurée - valeur de base) / tolérance, où les valeurs de base et les valeurs de tolérance sont, ainsi que cela a été défini précédemment, des valeurs définies par le médecin ou fonction de l'historique.

Les probabilités définissant les fonctions de transition T ont quant à elles été déterminées empiriquement.

Elle intervient au moment du calcul de la distribution de probabilité sur les cinq états modélisés par le modèle défini pour le suivi de l'hydratation.

Soit b cette distribution de probabilité sur S. b(s,t) est la probabilité que le patient soit dans l'état s à l'instant t. On peut estimer la probabilité que le patient soit dans l'état s' à l'instant t+1, connaissant l'observation o(t+1) et l'action a(t) par la formule :

$$p(s'|b,a,o) = \frac{p(o|s',a,b)p(s'|a,b)}{p(o|a,b)} = \frac{p(o|a,s')\sum_{s \in S} p(s'|a,s)b(s)}{p(o|a,b)}$$

avec $p(o|a,b) = \sum_{s' \in S} p(o|a,s') \sum_{s \in S} p(s'|s,a)b(s)$ et $p(o|a,s) = \prod_{i \in I} p(v_i|s)$

Les actions A et la fonction de récompense R sont optionnelles.

**Traitement mis en oeuvre sur les éléments ainsi définis**

Avec le modèle qui vient d'être décrit, le traitement mis en oeuvre par le serveur 2 qui reçoit les informations du patient est le suivant.

Une fois les valeurs $V_{i,t}$ des différents paramètres physiologiques i acquises pour le temps t, le vecteur d'observation constitué par ces différentes valeurs est traité pour déterminer les probabilités de se trouver dans l'état s et ce pour chacun des états.

Par exemple, la probabilité que l'hypotension orthostatique se trouve dans l'état se calcule à partir de l'équation suivante :

$$p(v_{i,t} | normal) = 5\% * s - \left( \frac{v_{i,t} - 0,5}{1,2} \right) + 83\% * cloche \left( \frac{v_{i,t} - 0,5}{1,2} \right) + 12\% * s + \left( \frac{v_{i,t} - 0,5}{1,2} \right)$$

pour i = hypotension orthostatique,
S- la sigmoïde définissant « inférieur-a-la-norme; » (figure B),
S+ la sigmoïde définissant « supérieur à la norme$_i$ » (figure C),
cloche la gaussienne définissant « conforme à la norme$_i$ » (figure A).

Les valeurs de 5%, 83% et 12% correspondent aux probabilités d'observer que cette hypotension orthostatique est inférieure à la norme, conforme à la norme ou supérieure à la norme, ces probabilités étant déterminées en fonction de l'historique du patient.

Les valeurs de 0,5 et 1,2 correspondent à celles de la référence normale et de la tolérance pour ce paramètre.

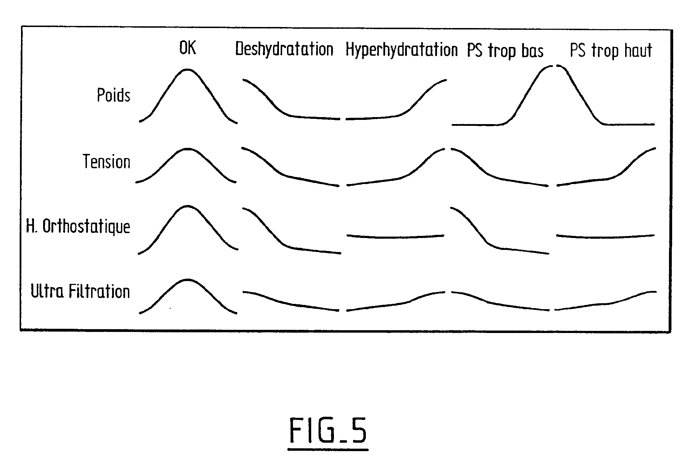

La figure 5 présente les différentes courbes de modélisation obtenues de cette façon pour les quatre paramètres physiologiques considérés et les cinq états du modèle.

Une fois ces probabilités déterminées, on en déduit la probabilité de se trouver dans un état donné compte tenu de l'ensemble des valeurs mesurées pour les paramètres physiologiques.

L'information ainsi déterminée est ensuite convertie pour être présentée au médecin.

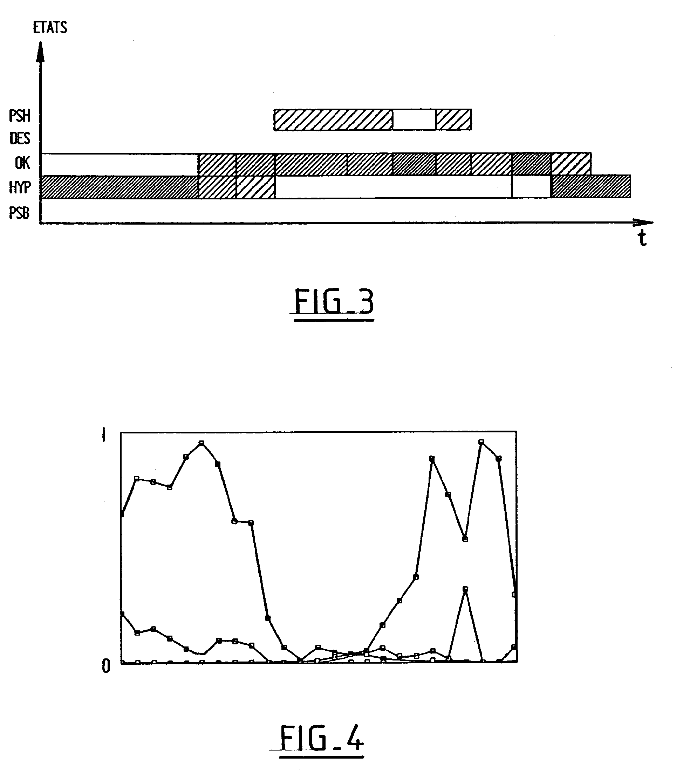

Par exemple, ainsi qu'illustré sur la figure 3, cette présentation peut se faire en fonction de différents niveaux de gris pour les différents états considérés.

Elle peut également être présentée sous une forme numérique, ainsi que l'illustre la figure 4. Sur le graphe de cette figure 4 est représentée une pluralité de courbes qui représente chacune l'évolution en fonction du temps des probabilités de se trouver dans l'état normal pour l'un des paramètres i.

Dans chacun des cas, l'information est présentée de façon temporelle.

On notera que le médecin a la possibilité de modifier les profils de niveaux de gris ou de valeurs de probabilité qui lui sont présentés pour les différents états, s'il estime que le diagnostic ne correspond pas au sien.

Supposons par exemple que la tendance qui apparaît au milieu du graphique (le Poids-Sec est certainement trop bas) soit une fausse alerte, et que le malade reste hyperhydraté tout le temps. Le médecin va simplement 'saisir' la courbe par les 'poignées' (les points encadrés) et les déplacer en vue de les remettre à la place qu'elle devrait occuper. Le résultat de ce processus est un nouveau profil, mais ce dernier n'a plus aucun lien avec les données saisies par le patient.

L'algorithme va donc ensuite essayer d'apprendre le lien entre la consigne qu'il vient de recevoir, et les données dont il dispose afin de proposer une solution viable. Celle-ci doit être aussi proche que possible de la consigne fournie par le médecin, mais elle doit être engendrée par le modèle markovien sous-jacent. De cette façon, on récupère un lien entre les données fournies par le patient et le profil. Cela conduit naturellement à des compromis.

On notera que le traitement d'adaptation consiste en des modifications des valeurs déterminées pour les probabilités $p_i(o/s)$, lesquelles modifications sont calculées de façon à minimiser l'erreur entre la nouvelle solution proposée par le médecin et la solution fournie par le modèle.

On notera que ce traitement revient à déterminer 40 paramètres pour l'ensemble des états sous la contrainte de minimiser l'erreur précitée.

En effet, les fonctions $f_{s,o}$ étant données, seuls les paramètres $t_i(o/s)$ sont à déterminer.

Par conséquent ceci revient à déterminer 12=3x4 valeurs de probabilités pour chaque état, sous les contraintes $p_i(\text{inférieur-a-la-norme}_i\ s) + p_i(\text{conforme}_i | s) + p_i(\text{supérieur-a-la-norme}_i | s) = 1$, soit au total 8 inconnues à déterminer par état.

On notera également qu'en variante, il peut être prévu que les moyens d'interface permettent au médecin de modifier directement les courbes de modélisations du type de celles de la figure 5.

**Revendications**

1. Système de suivi de patients soumis à une dialyse à domicile comportant des moyens d'interface chez le patient, des moyens d'interface chez un médecin, un serveur relié par un réseau aux moyens d'interface chez le patient et aux moyens d'interface chez le médecin, ledit serveur recevant des moyens d'interface chez le patient des valeurs de paramètres physiologiques mesurées par le patient ou par des capteurs et comportant des moyens pour mémoriser un historique, correspondant à une certaine période de temps, sur les valeurs de paramètres physiologiques qui lui sont transmises, ledit serveur comportant en outre des moyens pour mettre en oeuvre sur les valeurs de paramètres physiologiques transmises par le patient un traitement en vue de leur présentation et de la présentation de leur évolution sur les moyens d'interface chez le médecin,
lesdits moyens mettant en oeuvre sur les dernières valeurs de paramètres physiologiques qui sont transmises au serveur un traitement comparant l'état défini par ces différentes valeurs de paramètres physiologiques à des états d'alerte définis en fonction de l'historique mémorisé pour le patient et comportant des moyens pour transmettre les résultats de ce traitement aux moyens d'interface chez le médecin, **caractérisé en ce que** les moyens de traitement comprennent en outre des moyens pour calculer une probabilité d'observer une valeur donnée pour un paramètre physiologique donné et un temps donné, en supposant connu un état dans lequel se trouve le patient, de la manière suivante :

$$P(v_{i,t}|s) = \sum_{o \in O(i)} f_{s,o}(v_{i,t}) p_i(o|s)$$

où

$v_{i,t}$ est la valeur observée pour le paramètre physiologique $i$ à un instant $t$,
$O(i)$ est l'ensemble des observations possibles pour le paramètre $i$,
$f$ est une fonction qui donne une pondération comprise entre 0 et 1, qui dépend de la valeur du paramètre physiologique $i$ pour un état donné $s$ et dont les paramètres sont définis par le médecin ou dépendent de l'historique mémorisé pour le patient,
$p_i(o|s)$ est la probabilité d'observer o pour le paramètre $i$ en supposant que l'état du patient est $s$ ,

**en ce que** le calcul de probabilité est effectué pour différents états possibles pour le patient, et
**en ce que** les probabilités ainsi calculées pour les différents états possibles pour le patient sont transmises aux moyens d'interface chez le médecin.

2. Système selon la revendication 1, **caractérisé en ce que** l'ensemble $O(i)$ des observations possibles pour un paramètre $i$ comprend les observations suivantes : conforme, inférieur à la norme, supérieur à la norme.

3. Système selon la revendication 2, **caractérisé en ce que** les paramètres physiologiques i comprennent au moins l'un des paramètres suivants : poids, tension, tension orthostatique, bilan des poches.

4. Système selon la revendication 3, **caractérisé en ce que** les moyens de traitement comprennent des moyens pour comparer la différence entre le poids du patient mesuré et un poids sec fixé par le médecin avec des valeurs seuils inférieures ou supérieures, comparer la différence entre la dernière tension transmise et une tension moyenne calculée sur l'historique mémorisé pour le patient à des valeurs seuil, comparer la différence entre les tensions du patient lorsqu'il est couché et lorsqu'il est debout avec des valeurs seuils, comparer la différence entre le volume drainé par les poches du patient et un volume drainé moyen déterminé sur l'historique mémorisé pour le patient avec une valeur normale, et en déduire pour chaque paramètre $i$ une observation correspondante $o(i)$.

5. Système selon l'une des revendications 1 à 4, **caractérisé en ce que** la fonction $f$ dépend du rapport

fonction $f$ dépend du rapport

(valeur mesurée pour $i$ $-$ valeur de base pour $i$ )/ tolérance

où les valeurs de base et les valeurs de tolérance pour le paramètre physiologique $i$ sont définies par le médecin ou sont fonction de l'historique mémorisé pour le patient.

6. Système selon l'une des revendications précédentes, **caractérisé en ce que** le serveur comporte des moyens pour, lorsqu'un patient cherche à se connecter, identifier ledit patient, interrompre la communication et se connecter aux moyens d'interface dudit patient.

7. Système selon l'une des revendications précédentes, **caractérisé en ce que** les moyens d'interface chez le patient ne permettent à celui-ci de transmettre des informations à destination du médecin que lorsque le patient a rempli une fiche de paramètres quotidiens.

**8.** Système selon l'une des revendications précédentes, **caractérisé en ce que** les moyens d'interface chez le patient comportent des moyens pour contrôler la cohérence des valeurs de paramètres physiologiques mesurées et transmises par le patient.

**Patentansprüche**

**1.** System zum Betreuen von Patienten, die eine Heimdialyse erhalten, umfassend Schnittstellenmittel beim Patienten, Schnittstellenmittel bei einem Arzt, einen Server, der über ein Netzwerk mit den Schnittstellenmitteln beim Patienten und den Schnittstellenmitteln beim Arzt verbunden ist, wobei der Server von den Schnittstellenmitteln beim Patienten Werte von physiologischen Parametern, die von dem Patienten oder von Sensoren gemessen werden, empfängt und Mittel zum Speichern einer Vorgeschichte, die einem gewissen Zeitraum entspricht, über die Werte von physiologischen Parametern, die an diesen übertragen werden, umfasst, wobei der Server ferner Mittel zum Umsetzen einer Verarbeitung der Werte von physiologischen Parametern, die von dem Patienten übertragen werden, im Hinblick auf ihre Präsentation und die Präsentation ihrer Entwicklung an den Schnittstellenmitteln beim Arzt umfasst, wobei die Mittel an den neuesten Werten von physiologischen Parametern, die an den Server übertragen werden, eine Verarbeitung umsetzen, die den Zustand, der durch diese verschiedenen Werte von physiologischen Parametern definiert wird, mit Warnzuständen, die in Abhängigkeit von der Vorgeschichte definiert werden, die für den Patienten gespeichert ist, vergleichen, und Mittel zum Übertragen der Ergebnisse dieser Verarbeitung an die Schnittstellenmittel beim Arzt umfassen,
**dadurch gekennzeichnet, dass** die Verarbeitungsmittel ferner Mittel umfassen, um eine Wahrscheinlichkeit der Beobachtung eines bestimmten Wertes für einen bestimmten physiologischen Parameter und eine bestimmte Zeit, unter der Annahme, dass ein Zustand, in dem sich der Patient befindet, bekannt ist, wie folqt zu berechnen:

$$P\left(v_{i,t}|s\right) = \sum_{o \in O(i)} f_{s,o}\left(v_{i,t}\right) p_i(o|s)$$

wobei

$v_{i,t}$ der Wert ist, der für den physiologischen Parameter $i$ zu einem Zeitpunkt t beobachtet wird,
$O(i)$ die Menge der Beobachtungen, die für den Parameter $i$ möglich sind, ist,
$f$ eine Funktion ist, die eine Gewichtung zwischen 0 und 1 erteilt, die von dem Wert des physiologischen Parameters $i$ für einen bestimmten Zustand s abhängt, und deren Parameter durch den Arzt definiert werden, oder von der für den Patienten gespeicherten Vorgeschichte abhängig sind,
$p_i(o|s)$ die Wahrscheinlichkeit der Beobachtung von o für den Parameter $i$ ist, unter der Annahme, dass der Zustand des Patienten s ist,

dass die Wahrscheinlichkeitsberechnung für verschiedene Zustände, die für den Patienten möglich sind, erfolgt, und dass die somit berechneten Wahrscheinlichkeiten für die verschiedenen Zustände, die für den Patienten möglich sind, an die Schnittstellenmittel beim Arzt übertragen werden.

**2.** System nach Anspruch 1, **dadurch gekennzeichnet, dass** die Menge $O(i)$ der möglichen Beobachtungen für einen Parameter i die folgenden Beobachtungen umfasst: normal, niedriger als normal, höher als normal.

**3.** System nach Anspruch 2, **dadurch gekennzeichnet, dass** die physiologischen Parameters i mindestens einen der folgenden Parameter umfassen: Gewicht, Blutdruck, orthostatischer Blutdruck, Bilanz der Beutel.

**4.** System nach Anspruch 3, **dadurch gekennzeichnet, dass** die Verarbeitungsmittel Mittel umfassen, um die Differenz zwischen dem gemessenen Gewicht des Patienten und einem Trockengewicht, das vom Arzt festgelegt wird, mit niedrigeren oder höheren Schwellenwerten zu vergleichen, um die Differenz zwischen dem letzten übertragenen Blutdruck und einem durchschnittlichen Blutdruck, der anhand der Vorgeschichte berechnet wird, die für den Patienten gespeichert wird, mit Schwellenwerten zu vergleichen, um die Differenz zwischen den Blutdrücken des Patienten, wenn er liegt oder wenn er steht, mit Schwellenwerten zu vergleichen, um die Differenz zwischen dem abgenommenen Volumen der Beutel des Patienten und einem durchschnittlichen abgenommenen Volumen, das anhand der Vorgeschichte bestimmt wird, die für den Patienten gespeichert wird, mit einem normalen Wert zu vergleichen, und um davon für jeden Parameter i eine entsprechende Beobachtung o(i) abzuleiten.

**5.** System nach einem der Ansprüche 1 bis 4, **dadurch gekennzeichnet, dass** die Funktion *f* von dem Verhältnis

$$\texttt{(Messwert für } i \texttt{ - Basiswert für } i \texttt{) / Toleranz}$$

abhängt, wobei die Basiswerte und die Toleranzwerte für den physiologischen Parameter *i* vom Arzt definiert werden oder von der Vorgeschichte, die für den Patienten gespeichert wird, abhängig sind.

**6.** System nach einem der vorhergehenden Ansprüche, **dadurch gekennzeichnet, dass** der Server Mittel umfasst, um den Patienten zu identifizieren, wenn der Patient versucht sich anzumelden, um die Verbindung abzubrechen und um sich an die Schnittstellenmittel des Patienten anzuschließen.

**7.** System nach einem der vorhergehenden Ansprüche, **dadurch gekennzeichnet, dass** die Schnittstellenmittel beim Patienten diesem nur erlauben, Informationen an den Arzt zu übertragen, wenn der Patient ein Formular mit täglichen Parametern ausgefüllt hat.

**8.** System nach einem der vorhergehenden Ansprüche, **dadurch gekennzeichnet, dass** die Schnittstellenmittel beim Patienten Mittel umfassen, um die Widerspruchsfreiheit der Werte der physiologischen Parameter, die von dem Patienten gemessen und übertragen werden, zu überprüfen.

**Claims**

**1.** A system for monitoring patients undergoing home dialysis, comprising interface means at the patient's home, interface means at a doctor's surgery, a server connected via a network to the interface means at the patient's home and to the interface means at the doctor's surgery, said server receiving from the interface means at the patient's home values of physiological parameters measured by the patient or by sensors, and comprising means for storing a history corresponding to a certain period of time regarding the values of physiological parameters which are transmitted thereto, said server further comprising means for implementing processing of the values of physiological parameters transmitted by the patient with a view to presenting them and presenting the development thereof on the interface means at the doctor's surgery,
said means implementing processing of the latest values of physiological parameters being transmitted to the server by comparing the state defined by such different values of physiological parameters with alert states defined depending on the history stored for the patient, and comprising means for transmitting the results of such processing to the interface means at the doctor's surgery,
**characterized in that** the processing means further include means for calculating a probability of observing a given value for a given physiological parameter and a given time, assuming a state is known, in which the patient is, as follows:

$$P\big(v_{i,t}|s\big) = \sum_{o \in O(i)} f_{s,o}\big(v_{i,t}\big)\, p_i\big(o|s\big)$$

wherein

$v_{i,t}$ is the value observed for the physiological parameter *i* at a time *t*,
*O(i)* is the set of the observations possible for the parameter *i*,
*f* is a function giving a weighting between 0 and 1 which depends on the value of the physiological parameter *i* for a given state s and the parameters of which are defined by the doctor or depend on the history stored for the patient,
$p_i(o|s)$ is the probability of observing o for the parameter *i* assuming the patient's state is *s*,

**in that** the probability calculation is performed for different possible states for the patient, and
**in that** the probabilities thus calculated for the different states possible for the patient are transmitted to the interface means at the doctor's surgery.

**2.** The system according to claim 1, **characterized in that** the set *O(i)* of the possible observations for a parameter *i* include the following observations: normal, lower than normal, higher than normal.

**3.** The system according to claim 2, **characterized in that** the physiological parameters *i* include at least one of the following parameters: weight, blood pressure, orthostatic blood pressure, bag status.

**4.** The system according to claim 3, **characterized in that** the processing means include means for comparing the difference between the measured weight of the patient and a dry weight established by the doctor to lower or upper threshold values, for comparing the difference between the last blood pressure transmitted and an average blood pressure calculated based on the history stored for the patient to threshold values, for comparing the difference between the blood pressures of the patient when he or she is lying down or when he or she is standing up to threshold values, for comparing the difference between the volume drained by the bags of the patient and an average drained volume determined based on the history stored for the patient to a normal value, and for deriving therefrom for each parameter *i* a corresponding observation *o(i)*.

**5.** The system according to any of claims 1 to 4, **characterized in that** the function *f* depends on the ratio

```
(value  measured  for  i  -  base  value  for  i)  /
tolerance
```

wherein the base values and the tolerance values for the physiological parameter *i* are defined by the doctor or are a function of the history stored for the patient.

**6.** The system according to any of the preceding claims, **characterized in that** the server comprises means, when the patient is trying to log in, for identifying the patient, for disconnecting the communication, and for connecting to the interface means of said patient.

**7.** The system according to any of the preceding claims, **characterized in that** the interface means at the patient's home only allow him or her to transmit information to the doctor when the patient has filled in a form with daily parameters.

**8.** The system according to any of the preceding claims, **characterized in that** the interface means at the patient's home include means for checking consistency of the values of physiological parameters measured and transmitted by the patient.

FIG_1

FIG_2

## FIG.3

## FIG.4

|  | OK | Deshydratation | Hyperhydratation | PS trop bas | PS trop haut |
|---|---|---|---|---|---|
| Poids | | | | | |
| Tension | | | | | |
| H. Orthostatique | | | | | |
| Ultra Filtration | | | | | |

## FIG.5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2717332

- WO 9628086 A

**Littérature non-brevet citée dans la description**

- **KOENIG, R. G. SIMMONS.** Unsupervised Learning of Probabilistic Models for Robot Navigation. *IEEE ICRA,* vol. 96